# EUROPÄISCHE PATENTSCHRIFT

(11) **EP 0 553 770 B1**
(45) Veröffentlichungstag und Bekanntmachung des Hinweises auf die Patenterteilung: **26.08.1998**
(21) Anmeldenummer: 93101143.1
(22) Anmeldetag: 26.01.1993
(51) Int. Cl.: G01N 33/543, G01N 33/558, G01N 33/78

(54) **Analyseelement für Immunoassays**
Analytical element for immunoassays
Elément analytique pour les essais immunologiques

(30) Priorität: 31.01.1992 DE 4202850
(43) Veröffentlichungstag der Anmeldung: 04.08.1993
(73) Patentinhaber: BOEHRINGER MANNHEIM GMBH, 68298 Mannheim (DE)
(72) Erfinder: Goerlach-Graw, Ada, Dr., W-6714 Weisenheim am Sand (DE); Nägele, Ulrich, Dr., W-6940 Weinheim (DE); Wilk, Hans-Erich, Dr., W-6141 Einhausen (DE); Graw, Reiner, W-6714 Weisenheim am Sand (DE)

(56) Entgegenhaltungen:
- EP-A- 0 344 578
- EP-A- 0 469 444
- EP-A- 0 469 445
- WO-A-91/15769
- DE-A- 4 037 724
- DE-C- 3 445 816
- FR-A- 2 355 290

## Beschreibung

Die Erfindung betrifft ein Analysenelement zur Bestimmung eines Analyten in einer Probenflüssigkeit nach dem Prinzip eines heterogenen Immunoassays bestehend aus einem porösen kapillaraktiven Trägermaterial mit einer Reaktionszone und mindestens zwei Detektionszonen. Weiterhin betrifft die Erfindung ein Verfahren zur Bestimmung eines Analyten in einer Probenflüssigkeit nach dem Prinzip eines heterogenen Immunoassays unter Verwendung dieses Analysenelementes.

Auf dem Gebiet der medizinischen Diagnostik ist die Zahl der in physiologischen Probenflüssigkeiten zu bestimmmenden Substanzen immens angewachsen. Zur Bestimmung dieser Substanzen oder Analyten kommen immunologischen Nachweisverfahren eine zunehmende Bedeutung zu. Hervorzuheben sind dabei heterogene Immunoassays, bei denen normalerweise ein analytspezifischer bioaffiner Bindungspartner an einer Festphase gebunden vorliegt. Allgemein wird dabei zwischen kompetitiven und Immunoassays und Sandwich-Immunoassays unterschieden.

Bei kompetitiven Immunoassays konkurriert eine vorbestimmte Menge eines markierten Analytderivates mit den zu bestimmenden Analytmolekülen um die Bindungstelle des festphasengebundenen Bindungspartners. Nach einer Inkubationsphase wird das nichtgebundene Material ausgewaschen und die Menge des markierten Analytderivates in der gebundenen oder in der freien Phase als Maß für die Menge des Analyten bestimmt.

Bei Sandwich-Immunoassays wird im allgemeinen nach der Bindung des Analyten an den festphasengebundenen analytspezifischen Bindungspartner ein zweiter analytspezifischer Bindungspartner, der eine meßbare Markierung trägt, im Überschuß zugegeben. Nach Auswaschen des nichtgebundenen Materials wird die Menge des markierten Reagenzes in der gebundenen Phase als Maß für die Analytmenge bestimmt.

Seit einiger Zeit wurden heterogene Immunoassays auf sogenannten Testträgern aus porösen oder faserigen Materialien vorgeschlagen. Die chromatographische Eigenschaft dieser Testträger dient dazu, gebundenes von nichtgebundenem markiertem Reagenz zu trennen. Ziel war es weiterhin, auf solchen Testträgern alle für die heterogene Nachweisreaktion des Analyten notwendigen Bindungspartner auf einem Testträger zu integrieren und so die Zahl der Reagenzdosierschritte zu reduzieren (vollständig integrierte Analysenelemente).

US-Patent 4,361,537; EP-A-0 291 194 oder EP-A-0 186 799 beschreiben chromatographische Teststreifen mit einer Analytaufgabezone und einer Detektionszone, die auf der Ebene des Testträgers voneinander getrennt sind. Die Detektionszone enthält einen immobilisierten Bindungspartner. Der Bindungspartner kann für den Analyten direkt spezifisch sein (z. B. ein immobilisierter Antikörper), oder er kann spezifisch für einen analytspezifischen Bindungspartner sein (z. B. Streptavidin, das spezifisch biotinylierte Analytantikörper oder biotinylierte Analytderivate bindet). Die für den Immunoassay notwendigen weiteren Bindungspartner sind zwischen Aufgabezone und Detektionszone auf verschiedenen separaten Zonen nacheinander aufgebracht, um eine vorzeitige Wechselwirkung der Reagenzien untereinander vor Analytaufgabe zu verhindern. Eine solche Wechselwirkung, beispielsweise von markiertem Analytderivat mit den Bindungsstellen des analytspezifischen Bindungspartners, ist insbesondere bei kompetitiver Testführung solange möglichst zu vermeiden, bis der Analyt ebenfalls um diese Bindungsstelle konkurrieren kann.

Um überprüfen zu können, ob die chromatographische Wanderung der markierten Bindungskomponente zur Detektionszone stattfindet, bzw. ob die Markierung noch funktionstüchtig ist, wurde zusätzlich vorgeschlagen, hinter der Detektionszone eine weitere Detektionszone als Kontrollzone anzubringen, auf der z. B. Antikörper gegen den freien markierten Bindungsparatner immobilisiert sind. In dieser Kontrollzone wird der freie markierte Bindungspartner nach Passieren der ersten Detektionszone gebunden. So soll eine positive Reaktionskontrolle ermöglicht werden.

Dieses Prinzip der Reaktionskontrolle wird auch in DE-A 4037724 verwirklicht. Daneben beschreibt DE-A 4037724 unter anderem chromatographische Teststreifen, bei denen mehrere Analyten parallel nachgewiesen werden können. Diese Mehrfach-Devices zeichnen sich durch eine zentrale Probenaufgabezone aus, an die mehrere Reaktionszonen grenzen, die wiederum in Kontakt mit Membranen mit Dochtwirkung stehen, in denen schließlich die Detektionsreaktionen stattfinden.

Der Nachteil, der in den obigen Schriften beschriebenen chromatographischen Teststreifen liegt darin, daß, bedingt durch die serielle Anordnung der verschiedenen Immunreagenzien es nur zu einer unvollständigen homogenen Durchmischung der im Flüssigkeitsstrom nacheinander gelösten Reagenzien kommt. Die Konzentrationsverhältnisse an und hinter der Flüssigkeitsfront ändern sich ständig und damit auch die Bindungsgleichgewichte der verschiedenen Bindungspartner. So können diese Teststreifen im allgemeinen nur für qualitative und halbquantitative Analysen eingesetzt werden. Ferner ist das Problem einer positiven Reaktionskontrolle nicht zufriedenstellend gelöst, da die markierten Bindungspartner vor Erreichen der Kontrollzone zuerst die Detektionszone passieren müssen und hier durch unspezifische Wechselwirkungen festgehalten werden können. In der Kontrollzone können nur die freien, in der Detektionszone nicht abgefangenen oder hängengebliebenen markierten Bindungspartner nachgewiesen werden, aber dies erst mit einiger Zeitverzögerung zum eigentlichen, in der Detektionszone angezeigten Analysenergebnis. Eine quantitative Korrektur des Analysenergebnisses über solche Kontrollzonen ist nicht möglich.

Solche Teststreifen sind daher insgesamt relativ ungenau und benötigen lange Chromatographiezeiten. Es fehlt an quantitativen Kontrollmöglichkeiten des Testergebnisses und die Kontrolle der Funktion der markierten Komponente ist nicht befriedigend gelöst.

Aufgabe der vorliegenden Erfindung ist es deshalb, die Nachteile von Analysenelementen des Standes der Technik zu beseitigen und ein vollständig integriertes chromatographisches Analysenelement zur Verfügung zu stellen, das genauere Testergebnisse in kürzerer Zeit liefert, wobei die Testergebnisse zudem überprüfbar und korrigierbar sind und das Funktionieren des markierten Bindungspartners gleichzeitig und zuverlässig kontrolliert werden kann.

Die Aufgabe wird gelöst durch ein Analysenelement, wie es in der Ansprüchen charakterisiert ist.

Gegenstand der Erfindung ist ein Analysenelement zur Messung eines Analyten nach dem Prinzip eines heterogenen Immunoassays bestehend aus einem chromatographiefähigen Trägermaterial, mit einer Reaktionzone, mindestens zwei räumlich von der Reaktionszone getrennten Detektionszonen und mit Saugzonen, die sich an der von der Reaktionszone entfernten Seite an die Detektionszonen anschließen, dadurch gekennzeichnet, daß auf der Reaktionszone analytspezifische und markierte Bindungspartner auf einer Anzahl löslicher, eng benachbarter, aber voneinander räumlich getrennter Kompartimente vorliegen, daß jede Detektionszone ein Bindungsreagenz für einen der auf der Reaktionszone vorliegenden Bindungspartner immobilisiert enthält, wobei mindestens eine Detektionszone ein Bindungsreagenz für den analytspezifischen unmarkierten Bindungspartner enthält, und die Detektionszonen um die Reaktionszone so angeordnet sind, daß sie der Reaktionszone benachbart sind.

In dem Analysenelement liegt das poröse, kapillaraktive Trägermaterial als flächenförmige Matrix vor. Die Fläche kann beispielsweise quadratisch, rechteckig oder kreisförmig ausgebildet sein. Möglich ist aber auch ein entsprechenden Flächensegment, wie dies zum Beispiel ein Teststreifen darstellt. Als Material für die saugfähige Matrix kommen alle porösen Materialien in Frage, die aufgrund von Kapillarkraft Flüssigkeit und darin gelöste Reagenzien parallel zur Trägermaterialebene transportieren können und die nicht nachteilig mit den eingesetzten Reagenzien wechselwirken oder reagieren. Solche porösen Trägermaterialien sind dem Fachmann bekannt. Beispielsweise können Papier, Cellulose, Nitrocellulose, gepreßte Fasern, wie z. B. Glasfasern, gesintertes Glas, Keramik oder Kunststoffe poröser oder faseriger Struktur mit hinreichend hydrophilen Eigenschaften eingesetzt werden. Besonders vorteilhaft kann Nitrocellulose eingesetzt werden.

Die Wahl der Porengröße des Materials hängt von den jeweiligen Reaktionsbedingungen ab. Als günstige Porengröße haben sich die Bereiche 0,1 - 5 µm, bevorzugt 0,45 - 1 µm, erwiesen. Die Matrixdicke liegt vorteilhafterweise zwischen 50 und 250 µm.

Die gesamte heterogene Immunnachweisreaktion wird mit folgenden Bindungspartnern durchgeführt:
Ein erster Bindungspartner ist permanent trägerfixiert auf der Membran immobilisiert, der erste Bindungspartner ist dabei nicht analytspezifisch, aber spezifisch für einen zweiten Bindungspartner ("immobilisierter, nicht-analytspezifischer Bindungspartner").
Ein zweiter Bindungspartner ist spezifisch an den ersten Bindungspartner bindefähig und ist andererseits analytspezifisch ("analytspezifischer Bindungspartner") aber trägt keine Markierung. Dieser Bindungspartner liegt in nicht-immobilisierter Form vor, daß heißt, er wird bei Flüssigkeitskontakt frei löslich. Bei Kontakt mit dem ersten immobilisierten Bindungspartner wird er an diesen gebunden und erst dann immobilisiert.
Ein dritter Bindungspartner ist die markierte Reaktionskomponente ("markierter Bindungspartner") über die das Analysenergebnis ermittelt wird. Dieser Bindungspartner ist ebenfalls nicht-immobilisiert, d. h., er ist ablösbar auf dem Trägermaterial aufgebracht.

Methoden der Markierung von Immunkomponenten und deren Detektion sind dem Fachmann vertraut. Bewährt haben sich Direktmarkierungen, die ohne weitere Reagenzien nachweisbar sind, wie Farbstoffmoleküle, Metallsole, Fluorophore, Luminophore oder z. B. Phycobiliproteine oder fluoreszierender Latex. Besonders vorteilhaft kann Fluoreszenzmarkierung eingesetzt werden.

Die Art des dritten, markierten Bindungspartners hängt von dem immunologischen Testprinzip ab, das angewandt wird. Ist beispielsweise der Analyt ein Antigen, so kann der freie markierte Bindungspartner bei Verwendung eines kompetitiven Testprinzips ein dem Antigen entsprechendes markiertes Antigen sein. Der zweite Bindungspartner ist in diesem Fall ein an den ersten Bindungspartner bindefähiger Antikörper. Markierter Bindungspartner und Antigen konkurrieren um die Bindestelle des zweiten Bindungspartners, wobei die Menge des gebundenen markierten Analytanalogen ein Maß für die Konzentration des Analyten darstellt.

Bei Verwendung des Sandwich-Prinzips kann der freie markierte Bindungspartner ein markierter analytspezifischer Antikörper sein. Zweiter Bindungspartner und markierter Bindungspartner binden das Analytantigen über verschiedene Epitope. Die Menge des gebundenen markierten Bindungspartners stellt ein Maß für die Konzentration des Analyten dar. Die Begriffe Antigen und Antikörper können in der oben gegebenen Beschreibung ausgetauscht werden.

Als erster Bindungspartner (immobilisiert und nichtanalytspezifisch) kann bevorzugt Streptavidin (oder Avidin) verwendet werden, das den zweiten analytspezifischen Bindungspartner aufgrund dessen Konjugation mit einem Biotinmolekül spezifisch binden kann. Möglich sind aber auch alle dem Fachmann bekannten weiteren spezifischen Bindepaare wie Zucker/Lectin, komplementäre Nukleotidsequenzen, Enzym/Cofaktor, Antikörper/Antigen usw.

Das poröse kapillaraktive Trägermaterial beinhaltet auf der Trägerebene mindestens drei räumlich voneinander abgegrenzte flächenförmige Zonen auf der Trägerebene, die aufgrund der in ihnen enthaltenen Immunreagenzien zu funktionalen Zonen werden.

Die Reaktionszone befindet sich bevorzugt in zentraler Position der Trägermaterialfläche. Die Reaktionszone ist dadurch charakterisiert, daß sich in ihr in einem gemeinsamen, abgegrenzten Bereich die beiden Bindungspartner analytspezifischer Bindungspartner und markierter Bindungspartner auf vielen eng benachbarten aber räumlich voneinander getrennten Kompartimenten auf einer Fläche befinden.

Die Reaktionszone stellt bevorzugt einen kreisförmigen Bereich dar, möglich sind aber auch andere Formate, wie z. B. ein rechteckiges Format, im Falle eines Teststreifens. Die Flächengröße der Reaktionszone richtet sich im wesentlichen nach der Menge der aufzubringenden Immunreagenzien. Bevorzugt ist der Bereich der Reaktionszone ein Teil des Trägermaterial selbst. Möglich ist aber auch, daß im Bereich der Reaktionszone zusätzlich eine kapillaraktive Schicht auf das Trägermaterial aufgebracht ist, die mit dieser in flüssigkeitstransportierendem Kontakt steht und aller Immunreagenzien enthält.

Der Begriff "Kompartiment" bezeichnet einen gegenüber den Dimensionen der Reaktionszone wesentlich kleineren, flächenförmigen abgegrenzten Teilbereich, der eine Reagenzspezies enthält. Ein Kompartiment kann aus einem Fleck oder mehreren überlappenden Flecken oder aus einer Linie bestehen.

Die Kompartimente sind dadurch räumlich voneinander getrennt, daß sie in sehr geringem Abstand nebeneinander in der Reaktionszone angeordnet sind, wobei die Kompartimente mit verschiedenen Reagenzien bevorzugt alternieren, das heißt, daß wechselweise Kompartimente verschiedener Reagenzien benachbart sind. Aus praktischen Gründen ist ein regelmäßiges Alternieren zweckmäßig, bei dem sich also Kompartimente verschiedener Reagenzien in einer oder auch in zwei Flächenrichtungen zyklisch wiederholen, sodaß sich ein regelmäßiges Linien- bzw. Punktmuster ergibt. In Ausnahmefällen kann aber auch ein alternierendes Muster ohne zyklische Wiederholung zweckmäßig sein. Idealerweise sollte der Abstand der Kompartimente unendlich klein sein. Auf der anderen Seite sollten sich die Kompartimente verschiedener Reagenzien in trockenem Zustand gerade nicht berühren.

Praktischerweise liegt der Abstand der äußeren Begrenzung verschiedener Kompartimente zwischen 10 µm und 1 mm, bevorzugt zwischen 30 µm und 250 µm, ganz besonders bevorzugt zwischen 40 µm und 100 µm. Die Breite der Kompartimentlinien bzw. der Durchmesser der Kompartimentflecken sollte vorteilhafterweise weniger als 2 mm betragen und liegt bevorzugt zwischen 50 µm und 1 mm. Diese Formatierung und Anordnung der Kompartimente wird im folgenden auch als Mikrokompartimentierung bezeichnet.

Die Reagenzien, die in den Kompartimenten der Reaktionszone enthalten sind, sind nicht-immobilisiert, d. h. sie haften zwar in trockenem Zustand auf oder in der ein Kompartiment bildenden Trägermaterialfläche sind aber in einer auf die Reaktionszone aufgegebenen Flüssigkeit gut löslich ("lösliche Kompartimente"). Es empfiehlt sich daher als Trägermaterialien solche Materialien auszuwählen, die keine oder nur geringe unspezifische Wechselwirkungen mit dem Analyt oder den in löslichen Kompartimenten enthaltenen Immunreagenzien eingehen. Beispiele dafür sind modifizierte Nylonmembrane wie Loprodyne^{R} oder andere hydrophile Membrane wie hydrophiles Durapore^{R} der Firma Millipore.

Das Aufbringen der Immunreagenzien in Kompartimenten muß durch solche Verfahren erfolgen, die geeignet sind, mehrere verschiedene Immunreagenzien auf porösen Membranen in eng begrenzten und eng benachbarten Bereichen gezielt und in reproduzierbarer Weise aufzubringen, wobei die Immunreagenzien in feuchtem Zustand des Trägermaterials eluierbar sein sollen.

Das Aufbringen von Immunreagenzien in dieser Weise wurde bisher noch nicht beschrieben.

Als geeignet haben sich eine große Zahl verschiedener Drucktechniken herausgestellt, wie sie für enzymatische Testelemente in FR-A 2355290 genannt werden. Dazu zählen Siebdrucktechnik, die aus der Computerdrucktechnik bekannten Techniken wie Ink-Jet in verschiedenen Variationen, Nadeldrucktechniken, Sprühtechniken wie Air-brush, "Charged drop"-Druck-techniken und andere.

Bei der Siebdrucktechnik wird durch ein feinmaschiges Raster zuerst eine Komponente auf das Trägermaterial aufgestrichen, dann das Raster verschoben und eine zweite und gegebenenfalls weitere Komponenten in die Lücken des ersten Rasters aufgestrichen. Die Abstände der Kompartimente liegen eher an der oberen für die Erfindung noch tolerierbaren Grenze.

Eine Applikation von Immunreagenzien über Nadelapplikation eignet sich ebenfalls in solchen Fällen eher, indem die Abstände der Kompartimente nicht besonders klein zu sein brauchen.

Durch eine feine hohle Nadel von 0,05 - 1 mm Innendurchmesser wird mit einer extakt dosierenden Pumpe Reagenzflüssigkeit auf die Membran aufgebracht. Die Strichstärke hängt davon ab, wie schnell die Nadel über die Membran bzw. die Membran unter der Nadel bewegt wird. Bevorzugt ist eine Applikation von Reagenzien mit nach oben stehender Nadelöffnung und darüber geführter Nadel.

Bevorzugtere Verfahren im Sinne der Erfindung, sind Verfahren, die einen kleineren Abstand der Kompartimente erlauben. Dazu zählt beispielsweise die Applikation von Immunreagenzien mit dem sogenannten Airbrush-Verfahren. Dies ist ein kontinuierliches Sprühverfahren, bei dem, von einem Gasstrom umhüllt, Mikrotropfen auf eine Oberfläche bevorzugt in Linien aufgebracht werden. Der Gasstrom dient zum Ablenken und Positionieren des Mikrotropfenstrahls. Bekannt ist dieses Verfahren zum Aufbringen von Analytlösungen auf die Startlinie von Dünnschichtplatten.

Es hat sich herausgestellt, daß die hierfür kommerziell erhältlichen Geräte (zum Beispiel CAMAG DC-Probenautomat III) geeignet sind, verschiedene Immunreagenzien mikrokompartimentiert auf eine poröse Matrix aufzubringen. Dabei werden typischerweise Mikrotropfen der Größe 10 nl -1000 nl, bevorzugt unter 100 nl, auf die Multifunktionszone des erfindungsgemäßen Analysenelementes in Punkt- oder Strichmustern, vorzugsweise in alternierender Folge miteinander unverträglicher Reagenzien aufgebracht. Die Dichte der Linien beziehungsweise Punkte ist dabei abhängig von der Saugfähigkeit des Trägermaterials und der Größe der Tropfen und liegt vorzugsweise zwischen 10 und 100 Linien pro cm² beziehungsweise 100 bis 10.000 Tropfen pro cm².

Im Sinne der Erfindung noch vorteilhafter sind die ursprünglich für Computerdrucker (Tintenstrahldrucker) entwickelten Ink-Jet-Techniken, weil sich mit diesen Verfahren noch kleinere Reagenzflüssigkeitsquanten aufbringen lassen. Unter den verschiedenen Methoden dieser Technologie ist die sogenannte Bubble-Jet-Technik besonders bevorzugt. Ink-Jet-Techniken können in kontinuierliche und diskontinuierliche Techniken eingeteilt werden. Beide Gruppen sind für die Erfindung geeignet.

Die Anwendung zweier spezieller Varianten der diskontinuierlichen Ink-Jet-Technik zum Aufbringen eines Reagenzes auf einen räumlich abgegrenzten flächigen Bereich wird in EP-A-119 573 und EP-A-268 237 (US-A-4,877,745) beschrieben. Ein solcher abgegrenzter Bereich kann zum Beispiel die Form eines Plus- oder Minuszeichens zu haben, um das Analysenresultat deutlicher beziehungsweise für den Laien verständlicher zu machen, oder er kann dazu dienen eine unmittelbaren Vergleich zwischen einem mit reagenzbeschichteten Teilbereich und einem reagenzfreien Teilbereich zu ermöglichen. In Bezug zur Ink-Jet-Technologie wird auf die oben genannten Schriften hier Bezug genommen. Die Ink-Jet-Technik zeichnet sich dadurch aus, daß sehr kleine Teilmengen einer Flüssigkeit in hoher Präzision auf eine Tragschicht appliziert werden können. Die Präzision bezieht sich dabei sowohl auf die exakte Positionierung des von dem Reagenztropfen erzeugten Punktes auf der Tragschicht, als auch auf das Reagenzvolumen. Die Tropfen können mit hoher Frequenz hintereinander ausgestoßen werden.

Mit Ink-Jet-Techniken können auch verschiedene Reagenzien auf kleinem Raum in eng benachbarten, aber räumlich voneinander getrennten Kompartimenten auf porösen Membranen appliziert werden, um ein erfindungsgemäßes Analysenelement bereitzustellen.

Zur Herstellung eines erfindungsgemäßen Analysenelementes liegt das Volumen eines von einem Ink-Jet-Düsenkopf ausgestoßenen Quantums Reagenzflüssigkeit typischerweise zwischen 2 und 2000 nl, bevorzugt zwischen 100 und 800 nl. Die Fläche des von einem solchen Quantums auf der Tragschicht erzeugten Punktes ist stark von der Reagenzflüssigkeit und der Tragschicht abhängig. Sie liegt zwischen 3000 µm² und 0,1 mm² bevorzugt zwischen 500 µm² und 0,2 mm². Die Reagenzflüssigkeitsquanten werden typischerweise mit einer Frequenz von mehr als 1000 s⁻¹, bevorzugt zwischen 1000 und 200.000 s⁻¹ ausgestoßen. Nähere Einzelheiten können der am 06.02.92 veröffentlichten deutschen Patentanmeldung Aktenzeichen P 4024544.6 entnommen werden.

In den oben angegebenen Schriften EP-A-119 573 und EP-A-268 237 wurden zum großflächigen Applizieren von Reagenzien lediglich spezielle Varianten der Ink-Jet-Technologie benutzt, bei denen das Volumen einer Düsenkammer in einer Variante mechanisch in der anderen Variante piezoelektrisch komprimiert wird, wenn ein Tropfen ausgestoßen werden soll.

Es hat sich herausgestellt, daß unter den Ink-Jet-Techniken die bisher noch nicht zum Applizieren von Reagenzien beschriebene Bubble-Jet-Technik auch vorteilhaft zur Herstellung des erfindungsgemäßen Analysenelementes benutzt werden kann.

Bei der ebenfalls von Computerdruckern bekannten Bubble-Jet-Technik wird ein Teilvolumen der Flüssigkeit in der Düsenkammer jeweils kurzfristig verdampft und expandiert, um ein Quantum der Flüssigkeit durch die Düse auszustoßen. Es sind dabei keine mechanisch beweglichen Teile mehr erforderlich, wodurch sich eine hohe Zuverlässigkeit ergibt. Ferner kann über einen breiten Viskositätsbereich der Flüssigkeit gearbeitet werden. Trotz der starken Erhitzung der Flüssigkeit in der Düsenkammer kommt es überraschenderweise zu keiner praktisch bedeutsamen Beschädigung von in der Flüssigkeit gelösten Immunreagenzien. Zur Kompartimentierung der verschiedenen Reagenzien auf dem erfindungsgemäßen Analysenelement mit Ink-Jet-Techniken wird vorteilhafterweise mit einem Mehrkanaldruckkopf gearbeitet, wie sie für den Farbdruck entwickelt worden sind. Der Druckkopf kann mit einem von einer Steuereinheit gesteuerten X-Y-Antrieb in beiden Flächenrichtungen der Multifunktionszone positioniert werden. Ansonsten können die für die Ink-Jet-Technik, insbesondere die für die Bubble-Jet-Technik bekannten konstruktiven Einzelteile verwendet werden, die der Literatur über diese Techniken entnommen werden können. Nähere Einzelheiten können auch der am 06.02.92 veröffentlichten deutschen Patentanmeldung Aktenzeichen P 4024545.4 entnommen werden.

Die Reaktionszone des erfindungsgemäßen Analysenelementes ist von mindestens zwei Detektionszonen umgeben. Unter einer Detektionszone wird ein räumlich abgegrenzter, von der Reaktionszone durch einen flüssigkeitstransportierenden Bereich des Trägermaterials getrennter Bereich verstanden, in dem ein spezifisches Bindungsreagenz für eines der aus der Reaktionszone chromatographierenden Immunreagenzien immobilisiert vorliegt und in dem eine markierte Immunreaktionskomponente nachgewiesen wird. Die Immobilisierung des Bindungsreagenzes kann durch dem Fachmann bekannte Methoden wie kovalente Bindung oder Adsorption auf oder in dem Trägermaterial erfolgen.

Unter einem spezifischen Bindungsreagenz kann dabei sowohl ein Bindungsreagenz für den analytspezifischen Bindungspartner (und dessen Immunkomplexe) verstanden werden, als auch ein Bindungsreagenz, das den markierten Bindungspartner spezifisch bindet. Als Bindereagenz, das einen analytspezifischen Bindungspartner bindet, kann beispielsweise vorteilhaft Streptavidin benutzt werden, das als Poly-streptavidin oder TRSA-Streptavidin auf der Detektionszone ganzflächig, beispielsweise mit einem Druckverfahren, immobilisiert wird und biotinylierte Antikörper oder biotinylierte Analytderivate sehr schnell und fest binden kann (siehe EP-A-0 344 578). Es können aber auch andere bioaffine Bindungspartner wie Lectin/Zucker, komplementäre Nucleotidsequenzen, Enzym/Co-faktor, Antikörper/Antigen usw. eingesetzt werden.

Ein Bindungsreagenz, das einen markierten Bindungspartner bindet, kann z. B. ein immobilisierter Antikörper sein, der gegen die freien markierten Bindungspartner gerichtet ist.

In einer Detektionszone, die ein Bindungsreagenz gegen den analytspezifischen Bindungspartner immobilisiert enthält, wird das eigentliche Meßergebnis detektiert ("Meß-Detektionszone"), das heißt, die Anzahl der mit einem Markierungsreagenz gekuppelten analytspezifischen Bindepaare wird in dieser Zone als Maß für die Menge des Analyten in der Probenflüssigkeit gemessen.

In einer Detektionszone, die ein Bindungsreagenz für einen freien markierten Bindungspartner enthält, wird die Menge des gewanderten markierten Bindungspartners detektiert. Eine solche Zone kann auch als "Kontroll-Detektionszone" bezeichnet werden, da in ihr die chromatographische Wanderung des markierten Bindungspartners aus der Reaktionszone und seine Funktion kontrolliert werden kann.

Erfindungswesentlich ist, daß die zwei oder mehr Detektionszonen um die Reaktionszone so angeordnet sind, daß sie dieser benachbart sind. Darunter wird verstanden, daß eine Anordnung Reaktionszone - Detektionszone - Detektionszone in einer Linie ausgeschlossen wird. Jede Detektionszone soll lediglich durch das kapillaraktive Trägermaterial von der Reaktionszone getrennt sein. Besonders vorteilhaft ist es, wenn alle Detektionszonen den gleichen Abstand zur Reaktionszone haben. Beispielsweise können auf einem Teststreifen mit einer zentralen Reaktionszone je eine Detektionszone zu beiden Seiten der Reaktionszone auf der Teststreifenoberfläche aufgebracht sein, bevorzugt in gleichem Abstand von der Reaktionszone.

Mehr als zwei Detektionszonen können auf einem flächigem Trägermaterial konzentrisch in verschiedenen Richtungen um die Reaktionszone angeordnet werden. Bei gleichem Abstand zur Reaktionzone sind die Detektionszonen somit kreisförmig um die Reaktionszone angeordnet.

Von den mindestens zwei Detektionszonen, die um die Reaktionszonen angeordnet sind, ist mindestens eine Detektionszone eine Meß-Detektionszone, die ein Bindungsreagenz für den analytspezifischen Bindepartner und dessen Komplexe enthält. Die weiteren Detektionszonen sind eine oder mehrere Meß-Detektionszonen und/oder eine oder mehrere Kontroll-Detektionszonen, die immobilisiertes Bindungsreagenz für die markierten Bindungspartner enthalten.

Hinter den Detektionszonen, an der von der Reaktionszone abgewandten Seite, schließt sich der Saugzonenbereich des Trägermaterials an. Der Saugzonenbereich des Trägermaterials dient dazu, durch Kapillarkräfte Probenflüssigkeit, Waschflüssigkeit und nicht in der Detektionszone fixierte Reagenzien aufzunehmen. Vorteilhafterweise kann diese Funktion des Trägermaterials noch durch das zusätzliche Anbringen eines besonders saugfähigen Materials im Saugzonenbereich unterstützt werden. Bewährt hat sich dafür beispielsweise Whatman 3 MM Papier.

Ein weiterer Gegenstand der Erfindung ist ein Verfahren zur Bestimung eines Analyten in einer Probenflüssigkeit nach dem Prinzip eines heterogenen Immunoassays unter Verwendung des erfindungsgemäßen Analysenelementes, das dadurch gekennzeichnet ist, daß Probenflüssigkeit auf die Reaktionszone aufgegeben wird, mit anschließend auf die Reaktionszone aufgegebener Waschflüssigkeit gelöste Reagenzien aus der Reaktionszone in Richtung zu den Detektionszonen chromatographiert werden, die dort gebundenen markierten Reaktionskomponenten gemessen werden, und die Messungen in verschiedenen Detektionszonen zur qualitativen Kontrolle und/oder quantitativen Verbesserung der Analytbestimmung benutzt werden.

Zur Durchführung einer Analytbestimmung auf dem erfindungsgemäßen Analysenelement wird die den Analyten enthaltende Probenlösung auf die Reaktionszone aufgebracht. Bevorzugt erfolgt die Aufgabe auf die Mitte dieser Zone und das Volumen der Probenflüssigkeit sollte so groß sein, daß die gesamte Zone von Flüssigkeit benetzt wird. Durch die Auftragung der Reagenzien in Mikrokompartimenten werden die Reagenzien bei Aufgabe der Probenflüssigkeit schnell und gleichmäßig gelöst und reagieren untereinander schnell in einer homogenen Reaktion. Nach beendeter Reaktion der Bindungspartner wird eine Waschflüssigkeit bevorzugt auf das Zentrum der Reaktionszone aufgegeben und die gelösten Reagenzien und Immunkomplexe im wesentlichen nach allen Seiten radial aus der Reaktionszone chromatographiert. Bei Teststreifen erfolgt der Reagenzientransport aufgrund der äußeren Begrenzung des Streifens im wesentlichen gleichmäßig in beiden Längsrichtungen der Streifens nach außen. Als Waschflüssigkeit werden für Immunreagenzen übliche Pufferlösungen benutzt.

Erreichen Bindungspartner Detektionszonen, die die ihnen entsprechenden Bindungsreagenzien immobilisiert enthalten, werden sie dort festgehalten, während die übrigen Reagenzien die Detektionszone passieren und von der Saugzone aufgenommen werden.

Mindestens eine Detektionszone enthält immobilisierte Bindungsreagenzien für den analytspezifischen Bindungspartner, so daß diese Meß-Detektionszone über die Messung der immobilisierten Markierung ein erstes Meßergebnis für die Menge des Analyten in der Probenlösung liefert. Enthält das Analysenelement eine oder mehrere weitere Meß-Detektionszonen, erhält man ein oder mehrere weitere Meßergebnisse, die mit dem ersten Meßergebnis bzw. untereinander verglichen werden können. Der große Vorteil einer solchen Doppelt- oder Mehrfachmessung mit dem erfindungsgemäßen Analysenelement liegt darin, daß die meisten Parameter der Testführung identisch sind. Beispielsweise bilden die Reaktionspartner in der Reaktionszone eine homogene Lösung, die sich radial nach außen gleichmäßig verteilt, sodaß die Ausgangskonzentration der Reaktionspartner für jedes Meßergebnis identisch ist. Lediglich Inhomogenitäten des Reagenzientransports von der Detektionszone zu den verschiedenen Meß-Detektionszonen haben im wesentlichen Einfluß auf die verschiedenen Meßergebnisse und lassen sich durch Doppelt- oder Mehrfachmessungen auf verschiedenen Meß-Detektionszonen leicht herausmitteln, wodurch ein genaueres Meßergebnis erhalten wird.

Wenn man zwei oder mehrere voneinander getrennte, aber identische Meß-Detektionszonen zur Verfügung hat, hat man zusätzlich die Möglichkeit, die Genauigkeit der Analytbestimmung noch weiter zu erhöhen. Verschiedene Meß-Detektionszonen können gleichzeitig mit unterschiedlichen Wellenlängen vermessen werden. Dies ist insbesondere dann sinnvoll, wenn Komponenten der Analytenlösung die Messung in der Meß-Detektionszone stören. Beispielsweise zeigen viele Serumbestandteile, die unspezifisch in der Meß-Detektionszone binden können, einen erhöhten Fluoreszenzhintergrund. Solche Störungen können durch Messung zweier Meß-Detektionszonen bei verschiedenen Wellenlängen erfaßt und mathematisch eliminiert werden.

Enthält das Analysenelement neben einer oder mehreren Meß-Detektionszonen noch eine oder mehrere Kontroll-Detektionszonen, so kann die Zuverlässigkeit und Genauigkeit der Messung weiterhin erhöht werden. Eine Kontroll-Detektionszone im Sinne der Erfindung zeigt an, daß freie markierte Bindungspartner von der Reaktionszone zur Detektionszone gewandert sind. Ferner zeigt sie an, ob und in welchem Maße das Markierungssystem dieser Bindungspartner noch funktionsfähig ist, das heißt, in welchem Maß die Antikörper, Fluorophore oder Enzyme (bei Enzymmarkierung) beispielsweise durch Alterungserscheinungen geschädigt sind. Die in einer Kontroll-Dektektionszone erhaltenen Ist-Meßwerte lassen sich mit einem Soll-Referenzwert vergleichen, und so das in der Meß-Detektionszone erhaltene Ergebnis mit einem entsprechenden Faktor korrigieren. Der Vorteil des erfindungsgemäßen Analysenelementes liegt darin, daß diese Kontrolle unabhängig und gleichzeitig zu der Analytmessung stattfindet. Das Kontroll-Meßergebnis kann nicht dadurch verfälscht werden, daß der Flüssigkeitsstrom zuerst eine andere Detektionszone passieren muß. Auch kommt es zu keiner zeitlichen Verzögerung zwischen Analyt-Meßergebnis und der qualitativen Bestätigung dieses Ergebnisses.

Mit dem erfindungsgemäßen Analysenelement kann ein schnelles und sehr genaues Analysenergebnis erhalten werden.

Bei Aufgabe der Probenflüssigkeit auf die Reaktionszone werden die in Kompartimenten aufgebrachten Reagenzien gleichmäßig gelöst und reagieren in einer sehr schnellen homogenen Reaktion. Die Chromatographie der Reagenzien zu den Detektionszonen benötigt nur kurze Chromatographiewege was eine schnelle und genaue Testführung ermöglicht. Ein erstes Analysenergebnis mit gleichzeitiger positiver Reaktionskontrolle kann innerhalb von 10 - 300 Sekunden erhalten werden. Dadurch, daß mehrere voneinander unabhängige Detektionszonen auf einem Analysenelement aufgebracht werden können, ist es möglich, die Genauigkeit des Analysenergebnisses weiter zu verbessern. Werden die verschiedenen Detektionszonen gleichzeitig apparativ ausgewertet, erhält man ohne weiteren zusätzlichen Zeitbedarf ein sehr präzises korrigiertes Analysenergebnis.

Die Zeichnungen geben beispielhaft Ausführungsformen des Analysenelementes wieder:

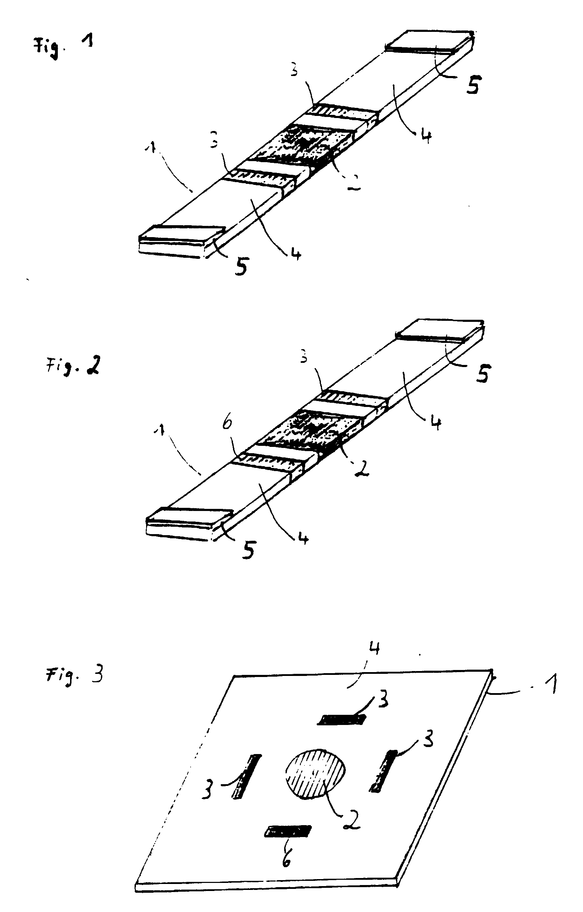

Figur 1 zeigt einen Teststreifen mit einem streifenförmigen Trägermaterial (1). In zentraler Position enthält das Trägermaterial eine Reaktionszone (2) (gleichzeitig Probenauftragszone). Im gleichen Abstand von der Reaktionszone befindet sich je eine mit Streptavidin beschichtete Meß-Detektionszone (3), mit der Reaktionszone (2) verbunden durch das kapillaraktive Material der Trägermatrix. Der Bereich der Trägermatrix hinter den Detektionszonen (3) dient als Absorptionsmittel (4) für überschüssige Flüssigkeit und kann von einem zusätzlichen absorptionsfähigen Material (5) unterstützt werden.
Figur 2 zeigt einen ähnlichen Teststreifen, bei dem eine streptavidinbeschichtete Meß-Detektionszone durch eine Kontroll-Detektionszone (6) ersetzt, die mit Antikörpern gegen einen im Test benutzten markierten Antikörper beschichtet ist.
Figur 3 zeigt ein Analysenelement mit einem Trägermaterial (1) und einer zentral aufgebrachten kreisförmigen Reaktionszone (2) mit mikrokompartimentierten Reagenzien. In gleichem Abstand um die Reaktionszone (2) befinden sich drei Meß-Reaktionszonen (3) und eine Kontroll-Detektionszone (6).

### Beispiel 1: Kompetitiver Immunoassay auf T4

### Reagenzien:

- A:: biotinylierter T4 -Antikörper (5 x 10⁻⁷M) in PBS-Puffer mit 10 Gew.% Trehalose und 0,2 Gew.% 8-Anilino-1-naphthalin-sulfonsäure (ANS).
- B:: T4-B-Phycoerythrin (1:1, 10⁻⁶M in obigem Puffer)
- C:: Poly-Streptavidin (polymerisiert mit Bis-hydroxysuccinimid, mittlere Molekülgröße < 70 nm, 5 mg/ml in obigem Puffer)

Reagenzien A und B wurden mit einem Hewlett-Packard-Paint-Jet-Mehrkanal-Drucker (Bubble-Jet) auf die Mitte einer 4 x 4 cm² großen Nitrocellulose-Membran (AES 98, Schleicher und Schüle) nach folgendem Linienmuster aufgebracht:

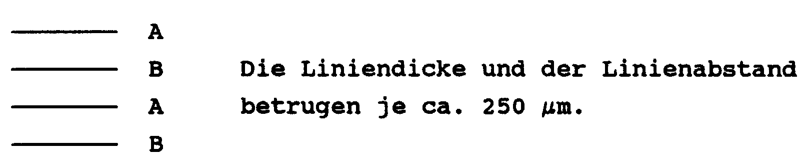

Die Auftragsmenge lag bei 0,5 µl Reagenz / cm².
Die Linien bedeckten ein Quadrat von 0,7 cm Kantenlänge. Das Quadrat stellt die Reaktionszone dar.

Reagenz C wurde mit einer dünnen Hohlnadel (Innendurchmesser 0,6 mm) in Form eines Quadrates mit der Seitenlänge 1,2 cm um das A/B-Linienquadrat aufgegeben. 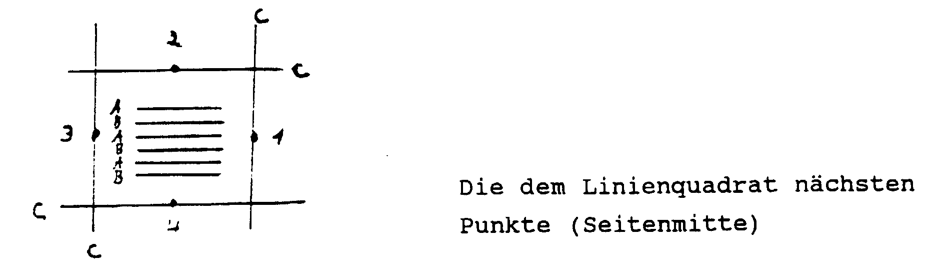 stellen 4 Streptavidin-Meß-Detektionszonen dar.

### Testdurchführung:

Die Membran wurde in einem Festphasenhalter eingebaut. 5 µl einer Analytprobe (Humanserum) aus einer Serumstandardreihe wurden auf die Mitte der Reaktionszone aufgegeben. Nach 3 Minuten wurden 3 x 15 µl Waschpuffer (PBS mit 0,1 Gew.% Tween 20) in 15 Sekunden-Abständen auf die Mitte der Reaktionszone aufgegeben. Nach weiteren 2 Minuten wurden 3 der 4 Detektionszonen an einem Hitachi 4010 bei Ex 515 nm, Em 580 nm vermessen.

Aus 5 Analysen sind die Meßwerte der drei Detektionszonen, die Mittelwerte dieser Werte und die Werte der Vermessung nur einer Detektionszone angegeben.

| T4-Konzentration nmol/l | Fluoreszenzeinheiten | | | Mittelwert X(1,2,3) | Detektionszone 1 |
|---|---|---|---|---|---|
| | Detektionszone 1 | Detektionszone 2 | Detektionszone 3 | | |
| 0 | 0,47 | 0,57 | 0,54 | 0,53 | 0,56 |
| 47,6 | 0,43 | 0,38 | 0,42 | 0,41 | 0,43 |
| 94 | 0,34 | 0,33 | 0,28 | 0,32 | 0,27 |
| 175 | 0,25 | 0,22 | 0,22 | 0,23 | 0,23 |
| 328 | 0,14 | 0,17 | 0,20 | 0,17 | 0,20 |

Der Mittelwert aus den Meßergebnissen dreier Detektionszonen liefert eine genaue gleichmäßige Kurve für verschiedene T4-Konzentrationen mit guten Abstufungen auch noch für T4-Konzentrationen über 100 mmol/l und somit reproduzierbare Ergebnisse.

Die Meßreihen mit nur einem Meßpunkt als Grundlage erzeugen dagegen stark streuende Kurven, die zum Teil bei T4-Konzentration von über 100 mmol/l nur kleine Abstufungen aufweisen.

## Patentansprüche

1. Analysenelement zur Bestimmung eines Analyten in einer Probenflüssigkeit nach dem Prinzip eines heterogenen Immunoassays bestehend aus einem chromatographiefähigen porösen Trägermaterial (1) mit einer Reaktionszone (2), die gleichzeitig Probenauftragszone ist, mindestens zwei räumlich voneinander getrennten Detektionszonen (3, 3, oder 3, 6) und Saugzonen (4), die sich an die Detektionszonen (3, 6) an den von der Reaktionszone (2) abgewandten Seiten anschließen, dadurch gekennzeichnet, daß auf der Reaktionszone (2) analytspezifische und markierte Bindungspartner auf einer Anzahl löslicher, räumlich voneinander getrennter Kompartimente vorliegen, wobei die Kompartimente einen Abstand von weniger als 1 mm voneinander haben, daß jede Detektionszone (3, 6) ein Bindungsreagenz für einen der auf der Reaktionszone (2) vorliegenden Bindungspartner immobilisiert enthält, wobei mindestens eine Detektionszone (3) ein Bindungsreagenz für den analytspezifischen unmarkierten Bindungspartner enthält, und die Detektionszonen (3, 6) so um die Reaktionszone angeordnet sind, daß sie der Reaktionszone (2) benachbart sind.

2. Analysenelement gemäß Anspruch 1, dadurch gekennzeichnet, daß zwei oder mehrere Detektionszonen (3, 6) im wesentlichen den gleichen Abstand von der Reaktionszone (2) haben

3. Analysenelement gemäß Anspruch 1 oder 2, dadurch gekennzeichnet, daß das Bindungsreagenz für den analytspezifischen unmarkierten Bindungspartner Streptavidin oder Avidin ist.

4. Analysenelement gemäß einem der Ansprüche 1 bis 3, dadurch gekennzeichnet, daß eine oder mehrere der Detektionszonen (6) ein Bindungsreagenz für den markierten Bindungspartner enthalten.

5. Analysenelement gemäß Anspruch 4, dadurch gekennzeichnet, daß das Bindungsreagenz für den markierten Bindungspartner ein Antikörper ist.

6. Analysenelement gemäß einem der Ansprüche 1 bis 5, dadurch gekennzeichnet, daß die Kompartimente der Reaktionszone (2) eine Ausdehnung in einer Flächenrichtung von weniger als 2 mm haben.

7. Analysenelement gemäß Anspruch 6, dadurch gekennzeichnet, daß die Ausdehnung in einer Flächenrichtung zwischen 50 µm und 1 mm beträgt.

8. Analysenelement gemäß einem der Ansprüche 1 bis 7, dadurch gekennzeichnet, daß die Kompartimente Linien bilden.

9. Analysenelement gemäß einem der Ansprüche 1 bis 8, dadurch gekennzeichnet, daß die Kompartimente Punkte bilden.

10. Analysenelement gemäß einem der Ansprüche 1 bis 9, dadurch gekennzeichnet, daß Kompartimente gleicher Reagenzien durch Kompartimente verschiedener Reagenzien voneinander getrennt sind.

11. Analysenelement gemäß einem der Ansprüche 1 bis 10, dadurch gekennzeichnet, daß die Kompartimente der Reaktionzone (2) über ein Ink-Jet-Verfahren aufgebracht sind.

12. Analysenelement gemäß Anspruch 11 dadurch gekennzeichnet, daß die Kompartimente der Reaktionszone (2) über ein Bubble-Jet-Verfahren aufgebracht sind.

13. Analysenelement gemäß einem der Ansprüche 1 bis 10, dadurch gekennzeichnet, daß die Kompartimente über Air-brush aufgebracht sind.

14. Verfahren zur Bestimmung eines Analyten in einer Probenflüssigkeit nach dem Prinzip eines heterogenen Immunoassays, dadurch gekennzeichnet, daß Probenflüssigkeit auf die Reaktionszone (2) des Analysenelementes gemäß einem der Ansprüche 1 bis 13 aufgegeben wird, gelöste Reagenzien durch auf die Reaktionszone (2) aufgegebene Waschflüssigkeit aus der Reaktionszone in Richtung zu den Detektionszonen (3, 6) chromatographiert werden, die dort gebundenen markierten Reaktionskomponenten gemessen werden, und die Meßergebnisse verschiedener Detektionszonen zur qualitativen Kontrolle und/oder quantitativen Verbesserung der Analytbestimmung benutzt werden.

15. Verfahren gemäß Anspruch 14, dadurch gekennzeichnet, daß die Meßergebnisse zweier oder mehrerer Detektionszonen (3), die ein Bindungsreagenz für den analytspezifischen Bindungspartner enthalten, gemittelt werden.

16. Verfahren gemäß Anspruch 14, dadurch gekennzeichnet, daß die Meßergebnisse zweier oder mehrerer Detektionszonen (3), die ein Bindungsreagenz für den analytspezifischen Bindungspartner enthalten, mit verschiedenen Meß-Verfahren erhalten werden.

17. Verfahren gemäß einem der Ansprüche 14 bis 16, dadurch gekennzeichnet, daß die Meßergebnisse einer oder mehrerer Detektionszonen (6), die ein Bindungsreagenz für den markierten Bindungspartner enthalten, zur positiven Reaktionskontrolle benutzt werden.

18. Verfahren gemäß einem der Ansprüche 14 bis 17, dadurch gekennzeichnet, daß die Meßergebnisse einer oder mehrerer Detektionszonen (6), die ein Bindungsreagenz für den markierten Bindungspartner enthalten, zur quantitativen Korrektur der Analytbestimmung benutzt werden.

19. Verfahren gemäß einem der Ansprüche 14 bis 18, dadurch gekennzeichnet, daß die Bestimmung des Analyten nach einem kompetitiven Immunassay durchgeführt wird.

## Claims

1. Analytical element for the determination of an analyte in a sample fluid using the principle of a heterogeneous immunoassay consisting of a chromatographic, porous carrier material (1) with a reaction zone (2) which is also the sample application zone, at least two spatially detection zones (3, 3 or 3, 6), and absorptive zones (4) provided at the sides of the detection zones (3, 6) facing away from the reaction zone (2), characterized in that the reaction zone (2) contains analyte-specific and labelled binding partners in a number of soluble compartments which are spatially separated but less than 1 mm apart, further characterized in that
each detection zone (3, 6) contains an immobilized binding reagent for a binding partner present on the reaction zone (2), and at least one of said detection zones (3) contains a binding reagent for the analyte-specific, unlabelled binding partner. and the detection zones (3, 6) are arranged around the reaction zone in such a way that they are adjacent to the reaction zone (2).

2. Analytical element according to claim 1, characterized in that two or more detection zones (3, 6) are essentially equidistant from the reaction zone (2).

3. Analytical element according to claim 1 or 2, characterized in that the binding reagent for the analyte-specific, unlabelled binding partner is streptavidin or avidin.

4. Analytical element according to one of the claims 1 to 3, characterized in that one or more of the detection zones (6) contain a binding reagent for the labelled binding partner.

5. Analytical element according to claim 4, characterized in that the binding reagent for the labelled binding partner is an antibody.

6. Analytical element according to one of the claims 1 to 5, characterized in that the compartments in the reaction zone (2) extend less than 2 mm in one surface direction.

7. Analytical element according to claim 6, characterized in that the compartments in the reaction zone (2) extend between 50 µm and 1 mm long in one surface direction.

8. Analytical element according to one of the claims 1 to 7, characterized in that the compartments form lines.

9. Analytical element according to one of the claims 1 to 8, characterized in that the compartments form dots.

10. Analytical element according to one of the claims 1 to 9, characterized in that compartments containing identical reagents are separated from each other by compartments containing different reagents.

11. Analytical element according to one of the claims 1 to 10, characterized in that the compartments of the reaction zone (2) are applied with an ink-jet technique.

12. Analytical element according to claim 11, characterized in that the compartments of the reaction zone (2) are applied with a bubble-jet technique.

13. Analytical element according to one of the claims 1 to 10, characterized in that the compartments are applied with an air-brush technique.

14. Process for the determination of an analyte in a sample fluid using the principle of a heterogeneous immunoassay, characterized in that sample fluid is applied to the reaction zone (2) of the analytical element according to one of the claims 1 to 13, wash fluid applied to the reaction zone (2) causes dissolved reagents to chromatograph out of the reaction zone towards the detection zones (3, 6), the labelled reaction components bound in said detection zones are measured, and the measured results from various detection zones are used as a qualitative control and/or for the quantitative improvement of the analyte determination.

15. Process according to claim 14, characterized in that the results from two or more detection zones (3) that contain a binding reagent for the analyte-specific binding partners are averaged.

16. Process according to claim 14, characterized in that the results from two or more detection zones (3) that contain a binding reagent for the analyte-specific binding partner are measured using different methods.

17. Process according to one of the claims 14 to 16, characterized in that the results measured with one or more detection zones (6) that contain a binding reagent for the labelled binding partner are used as the positive reaction control.

18. Process according to one of the claims 14 to 17, characterized in that the results measured with one or more detection zones (6) that contain a binding reagent for the labelled binding partner are used for the quantitative correction of the analyte determination.

19. Process according to one of the claims 14 to 18, characterized in that the analyte is determined using the principle of a competitive immunoassay.

## Revendications

1. Elément analytique pour la détermination d'un analyte dans un liquide d'échantillonnage suivant le principe d'un essai immunologique hétérogène, constitué d'un matériau de support (1) poreux apte à la chromatographie avec une zone de réaction (2), qui est en même temps la zone d'application de l'échantillon, au moins deux zones de détection (3, 3 ou 3, 6) séparées dans l'espace et zones d'aspiration (4) qui font suite aux zones de détection (3, 6) sur les côtés opposés à la zone de réaction (2), caractérisé en ce que dans la zone de réaction (2) se trouvent des partenaires de liaison marqués et spécifiques à l'analyte dans un certain nombre de compartiments solubles, séparés dans l'espace, les compartiments étant distants l'un de l'autre de moins de 1 mm, en ce que chaque zone de détection (3, 6) contient sous forme immobilisée un réactif de liaison pour un des partenaires de liaison se trouvant dans la zone de réaction (2), au moins une zone de détection (3) contenant un réactif de liaison pour le partenaire de liaison non-marqué spécifique à l'analyte, et en ce que les zones de détection (3, 6) sont disposées autour de la zone de réaction de telle façon qu'elles sont adjacentes à la zone de réaction (2).

2. Elément analytique selon la revendication 1, caractérisé en ce que deux ou plusieurs zones de détection (3, 6) se trouvent essentiellement à la même distance de la zone de réaction (2).

3. Elément analytique selon la revendication 1 ou 2, caractérisé en ce que le réactif de liaison pour le partenaire de liaison non-marqué spécifique à l'analyte est la streptavidine ou l'avidine.

4. Elément analytique selon l'une des revendications 1 à 3, caractérisé en ce qu'une ou plusieurs des zones de détection (6) contien(nen)t un réactif de liaison pour le partenaire de liaison marqué.

5. Elément analytique selon la revendication 4, caractérisé en ce que le réactif de liaison pour le partenaire de liaison marqué est un anticorps.

6. Elément analytique selon l'une des revendications 1 à 5, caractérisé en ce que les compartiments de la zone de réaction (2) présentent une dimension dans une direction superficielle de moins de 2 mm.

7. Elément analytique selon la revendication 6, caractérisé en ce que la dimension dans une direction superficielle se situe entre 50 µm et 1 mm.

8. Elément analytique selon l'une des revendications 1 à 7, caractérisé en ce que les compartiments forment des lignes.

9. Elément analytique selon l'une des revendications 1 à 8, caractérisé en ce que les compartiments forment des points.

10. Elément analytique selon l'une des revendications 1 à 9, caractérisé en ce que des compartiments de réactifs identiques sont séparés par des compartiments de réactifs différents.

11. Elément analytique selon l'une des revendications 1 à 10, caractérisé en ce que les compartiments de la zone de réaction (2) sont appliqués par un procédé ink-jet.

12. Elément analytique selon la revendication 11, caractérisé en ce que les compartiments de la zone de réaction (2) sont appliqués par un procédé bubble-jet.

13. Elément analytique selon l'une des revendications 1 à 10, caractérisé en ce que les compartiments sont appliqués par air-brush.

14. Procédé pour la détermination d'un analyte dans un liquide d'échantillonnage suivant le principe d'un essai immunologique hétérogène, caractérisé en ce que le liquide d'échantillonnage est appliqué sur la zone de réaction (2) de l'élément analytique selon l'une des revendications 1 à 13, en ce que les réactifs mis en solution par le liquide de lavage appliqué sur la zone de réaction (2) sont chromatographiés de la zone de réaction en direction des zones de détection (3, 6), en ce que les composantes réactionnelles marquées qui y sont liées sont mesurées, et en ce que les résultats de mesure de différentes zones de détection sont utilisés pour le contrôle qualitatif et/ou l'amélioration quantitative de la détermination de l'analyte.

15. Procédé selon la revendication 14, caractérisé en ce qu'on calcule la moyenne des résultats de mesure de deux ou de plusieurs zones de détection (3) qui contiennent un réactif de liaison pour le partenaire de liaison spécifique à l'analyte.

16. Procédé selon la revendication 14, caractérisé en ce que les résultats de mesure de deux ou de plusieurs zones de détection (3) qui contiennent un réactif de liaison pour le partenaire de liaison spécifique à l'analyte sont obtenus avec des procédés de mesure différents.

17. Procédé selon l'une des revendications 14 à 16, caractérisé en ce que les résultats de mesure de deux ou de plusieurs zones de détection (6) qui contiennent un réactif de liaison pour le partenaire de liaison marqué sont utilisés pour le contrôle positif de la réaction.

18. Procédé selon l'une des revendications 14 à 17, caractérisé en ce que les résultats de mesure de deux ou de plusieurs zones de détection (6) qui contiennent un réactif de liaison pour le partenaire de liaison marqué sont utilisés pour la correction quantitative de la détermination de l'analyte.

19. Procédé selon l'une des revendications 14 à 18, caractérisé en ce que la détermination de l'analyte est réalisée après un essai immunologique compétitif.
